# EUROPEAN PATENT SPECIFICATION

(11) **EP 3 169 284 B1**
(45) Date of publication and mention of the grant of the patent: **17.10.2018**
(21) Application number: 15738581.6
(22) Date of filing: 10.07.2015
(51) Int. Cl.: A61F 5/44

(54) **A DRAINABLE COLLECTION BAG**
ENTLEERBARER SAMMELBEUTEL
SAC COLLECTEUR VIDANGEABLE

(30) Priority: 16.07.2014 DK 201400389
(43) Date of publication of application: 24.05.2017
(73) Proprietor: Coloplast A/S, 3050 Humlebæk (DK)
(72) Inventor: LYSGAARD, Jes, 4622 Havdrup (DK)
(86) International application number: PCT/DK2015/050212
(87) International publication number: WO 2016/008494

(56) References cited:
- WO-A1-2007/115575
- WO-A1-2009/124324
- US-A1- 2003 073 962
- US-A1- 2010 217 214

## Description

### Field of the invention

The invention relates to a drainable collecting bag having improved closure.

### Background of the invention

Drainable collecting bags is often used as ostomy bags. In the case of ileostomy patients and colostomy patients with uncontrolled release of faeces of a more or less fluid consistence, the collecting bag has to be emptied rather frequently, and the closure device thus has to be easy to open and re-close after emptying and at the same time provide a reliable and tight seal in operation, i.e. between emptying the bag.

Such a bag typically has flat opposing sidewalls secured together along their edges and defining a cavity for receiving body waste material. One of the walls is provided with a stoma-receiving opening, and means are provided for securing the bag to a patient's abdomen so that waste discharge from the stoma is received in the cavity. At is lower end, the drainable bag has a discharge opening for draining waste material, usually provided at the end of a narrowed neck portion. Closure means is provided for maintaining the discharge opening in sealed condition until such time as waste material is to be drained from the bag. The closure means may take the form of a clamp or some device for securing the neck portion in upwardly-rolled condition. As will be appreciated, conventional wire ties or wraps have also been used for that purpose.

A drainable bag is reusable following periodic emptying of waste material, but cleaning is necessary prior to reuse so that effective sealing can be assured and odors emanating from the resealed bag can be avoided. Users often encounter difficulty and discomfort in unsealing, emptying, cleaning and resealing drainable bags because of the direct exposure to waste material and because the manipulations may require greater dexterity than a patient, particularly an elderly patient, can provide. Adding to the problem is the fact that residual amounts of solid and/or liquid waste matter at the lower end of a drainage bag tends to block or hold the walls of the bag together, making cleaning of the inside surfaces adjacent the discharge opening even more difficult.

US 2010/217214 discloses an arrangement for connecting faecal receiving bags together. The arrangement comprises plates for attachment to the plates of a collection bag. The plates are adapted to engage with the plates of a faecal bag, and the plates are provided with adhesive in order to provide a tight fit to the bag.

WO 2007/115575 discloses a collecting bag with a closure comprising two plates. The plates of the bag have substantially the same length as the discharge opening.

US 2003/073962 discloses a collecting bag with one plate at the outlet.

WO 2009/124324 discloses a collecting bag with an outlet bordered by two flaps.

### Brief description of drawings

Fig. 1a is a front elevational view of a drainable collection bag having a first and a second plate bordering the discharge opening and angled fastening strips.
Figure 1b is a cross section of the lower part of the drainable collection bag of Figure 1a.
Fig. 2 is a rear elevational view of the drainable collection bag of Fig. 1a.
Fig. 3 is a schematic front elevational view of the drainable collection bag with the neck portion folded into closed configuration and partly hidden behind the cover layer.
Fig. 4 is a schematic front elevational view of the drainable collection bag with the neck portion folded into closed configuration and fixed with the fastening strips.
Fig. 5 is a schematic front elevational view of the drainable collection bag with the neck portion unfolded and in open configuration and with the plates closed.
Fig. 6 and 7 are schematic front elevational views of the drainable collection bag with the neck portion unfolded and in open configuration and with the plates being inverted.
Fig. 8 is a front elevational view of the drainable collection bag where the content of the cavity is discharged.
Fig. 9 is a front elevational view of the drainable collection bag where the discharge opening is wiped clean after discharge of content.

### Summary of the Invention

The invention relates to a drainage collecting bag for human body wastes as defined in claim 1. Preferred embodiments are according to the dependent claims. A drainable collecting bag for human body wastes may comprise a front wall and a rear wall of flexible sheet material defining a cavity there between and which may define a downwardly extending neck portion terminating in a discharge opening. The discharge opening may be bordered with two plates that are configured to pivot 180 degrees to switch between an open configuration for emptying of the cavity and a closed configuration for liquid tight closure of the bag.

### Detailed Description of the Invention

In a first aspect, the invention relates to a drainable collecting bag for human body wastes comprising an adhesive wafer comprising a waste inlet opening, a front wall and a rear wall of flexible sheet material defining a cavity there between and which define a downwardly extending neck portion terminating in a discharge opening, a first and a second plate member, the first and the second plate members comprising at least a first edge portion and a second edge portion, the first plate member being attached to the front wall and the second plate member attached to the rear wall, the first edge of the first plate member and the first edge of the second plate member are facing each other and said discharge opening is extending between said edges, wherein the first and the second plates is configured to pivot 180 degrees around the first edge portion with respect to the front and the rear wall.

The neck portion is configured to be folded into a closed configuration by folding the neck portion around the plates and an open configuration where the neck portion is unfolded facilitating emptying the contents from the cavity.

By the first and the second plates being configured to be able to pivot 180 degrees around the first edge portion with respect to the front and the rear wall is meant that the first and the second plate are attached to the front and the rear wall at least along the first edge portion and that they are able to pivot freely around an axis corresponding to the first edge portion. Thus, the plates are able to pivot around the first edge portion without folding the neck portion.

The plates being able to pivot with respect to the neck portion enables them to be configured in a discharge configuration where the plates are folded back such that a first surface of the plates are facing the surface of the neck portion (front and rear wall). Thus, the first surfaces of the plates are facing each other's, with the neck portion in between. In this configuration the user may squeeze the content out of the bag using his fingers applying pressure to the neck portion and his fingers will be protected from contamination with the content of the bag by the plates. As the plates are not attached along the side edge of the neck portion, they can be slightly lifted in order to assure the entire content is squeezed out of the neck portion next to the discharge opening. If the plates are extending further than the width of the neck portion, the extended portion (tab member) may serve as a handle. When the bag is emptied, the discharge opening can easily be cleaned by wiping as only the first edges of the plates will be smudged. Cleaning will be easier as the only part of the bag that becomes dirty is the edges of the opening/plates, and these can easily be wiped clean.

When the bag is emptied and the closure is cleaned, the plates are inverted to the opposite configuration, by pivoting the plates 180 degrees with respect to the neck portion. The first edge of the plates serves as axis for the pivoting of the plates. Now the second surface of the first plate is facing the second surface of the second plate. In this configuration the plates may be placed overlying each other's and in direct contact, without the neck portion in between. Now the plates are pressed firmly together and the combined plates are folded towards the neck portion to lye against the walls of the neck portion. The flexible material of the plates squeezes together and at the same time the sidewalls of the neck portion stretches and the outlet is sealed. Folding is continued so the neck portion are wrapped around the combined plates to provide a tight and effective closing of the bag.

In embodiments, the first and the second plates are non-adhesive. By non-adhesive should herein be understood that the surface of the plates does not show adhesive properties. This facilitates easy cleaning of the plates.

The front and/or rear walls of the bag may be provided with a cover layer which is soft and pleasant to have against the skin. The cover layer may be attached along the edge potion of the bag for example by welding or adhesive. The cover layer may preferably not extend to the neck portion. The cover layer may be provided with a slit or in be a two-part system allowing the user to inspect the content of the cavity. The cover layer may be in the form of a non-woven material. The cover layer may be opaque or non-transparent in order to hide the content of the bag.

The neck portion may be rolled or folded at least one, such as two times around the plates to create a waterproof closure of the bag.

In order to avoid unintended unfolding of the neck portion, the bag may be provided with locking means.

The locking means may be in the form of hook-and-loop such as Velcro or it may be an adhesive, pressure studs or other suitable locking means. The locking means may be in the form of one or more parts being provided on one or both walls of neck portion or bag or the locking means may be located on fastening strips extending outwards from the neck portion. One part (such as the loops) may be provided on the fastening strips and the other part (such as the hooks) on the neck portion.

The locking means may be provided on fastening strips. The strips may be elongated strips extending from the side edges of the front and rear wall. The strips may be made from the same material as the front and rear wall and may constitute a part of the rear and front wall. The strips may be reinforced in order to provide extra strength or stiffness.

The fastening strips may be located parallel to the discharge opening or they may be located in an angle less than 90 degrees to the discharge opening. The discharge opening may define a slit between the plates, thus parallel to the first edge portion of the plates. The angle is defined as the angle to a line drawn along this slit, this line being substantially perpendicular to the vertical axis of the bag and neck portion.

The fastening strips may be mounted substantially parallel to the discharge opening or they may define an angle of approximately 45 degrees with the opening such as 75-15 degrees, 60-30 degrees, 55-35 degrees or even 50-40 degrees with the discharge opening.

The angled fastening strips provide better fixation of the folded/rolled neck portion by crossing over the folding.

The angled fastening strips may be pointing towards the cavity in the sense that the fastening strips are pointing /away from the discharge closure.

The collection bag may comprise a two-part fastening system for selectively and releasably holding the neck portion in upwardly folded condition; the system including a first fastener part located along the outer surface of one of the sidewalls of the neck portion and a second fastener part located on the fastening strips; the first and second fastener parts configured to be wrapped around the upwardly folded neck portion and to be brought into mutual engagement and attachment when the neck portion is in its fully folded condition.

In another respect, a drainable collection bag has a discharge opening that is placed in closed condition by folding the bag in a first direction and is placed in an open condition by unfolding the bag in a second, opposite direction. A two-part fastener is provided for selectively holding the discharge opening in the closed condition, and it includes a first part associated with the bag generally adjacent to or near the discharge opening and a second part associated with the bag spaced further from the discharge opening than the first part. More specifically, the second fastener part is spaced from the discharge opening to require folding the bag until the first fastener part comes into confronting relation with the second fastener part for engagement therewith.

The plates may be in the form of a sheet like material comprising at least a first and a second edge portion and a first and a second surface. The plates may be mirror images of each other's. The plates comprise a flexible material such as a polymer sheet or a closed cell foam sheet. The plates are stiffer than the walls of the bag.

The first edge portion of the plate members may have a length at least corresponding to the width of the discharge opening or it may have a length extending further than the width of the discharge opening.

The second edge portion may have the same length as the first edge portion. The second edge Portions may have a length extending further than the width of the discharge opening, thereby providing a part of the plates extending further than the width of the neck portion.

The first and the second edge portion of each plate may be connected by a sloping edge portion. The sloping edge portion may define an angle corresponding to the angle of the fastening strips or a mirror image of this angle. This enables a snug fit of the fastening strips around the folded neck portion and plates when the bag is in closed configuration and a better fixation of the folded neck portion even when it is exposed to pressure from a filled bag.

The sloping edge portion may define an angle of approximately 45 degrees with the discharge opening such as 75-15 degrees, 60-30 degrees, 55-35 degrees or even 50-40 degrees with the discharge opening.

The first and the second plate may be provided with a tab member. The tab member may constitute the part of the plates extending further than the width of the neck portion. The tab member may be designed for easy gripping with the fingers and may ease handling of the bag during emptying. The tabs may enable better hold and control of the opening and the bag during emptying of the bag when the content of the bag is squeezed out.

The first and the second edge portion of the plate members may be substantially parallel.

The first and the second plate members may be connected by at least two strap members. The strap members may be located on each side of the discharge closure. The strap members may be formed integrally with the plate members, said strap members and plate members forming a unit, for example being provided as a single molded unit.

The straps may be provided with indentations or a thinner area at the transition between the plates in order to facilitate easy bending of the strap when the plates are pivoted. The strap may be an integrated part of the plates, being cut or molded together.

The plates may be attached to the side walls of the bag by welding or adhesive or other suitable way. The plate may be attached in a way that enables at least one of the plates to pivot freely around the first edge. The walls may extend further over at least a part of the plate but in a way that allows the plate to pivot with respect to the wall to which it is attached.

The plates provide a handle as well as they offer protection for the user from contaminating his fingers with faeces during emptying of the bag.

A drainable collection bag has typically flat opposing sidewalls (front and rear walls) secured together along their edges and defining a cavity for receiving body waste material. One of the walls is provided with a stoma-receiving opening, and means are provided for securing the bag to a patient's abdomen so that waste discharge from the stoma is received in the cavity. At is lower end, the drainable bag has a discharge opening for draining waste material, provided at the end of a narrowed neck portion.

Other advantages and features of the invention will become apparent from the following specification when considered in view of the accompanying drawings.

### Detailed Description of the Drawing

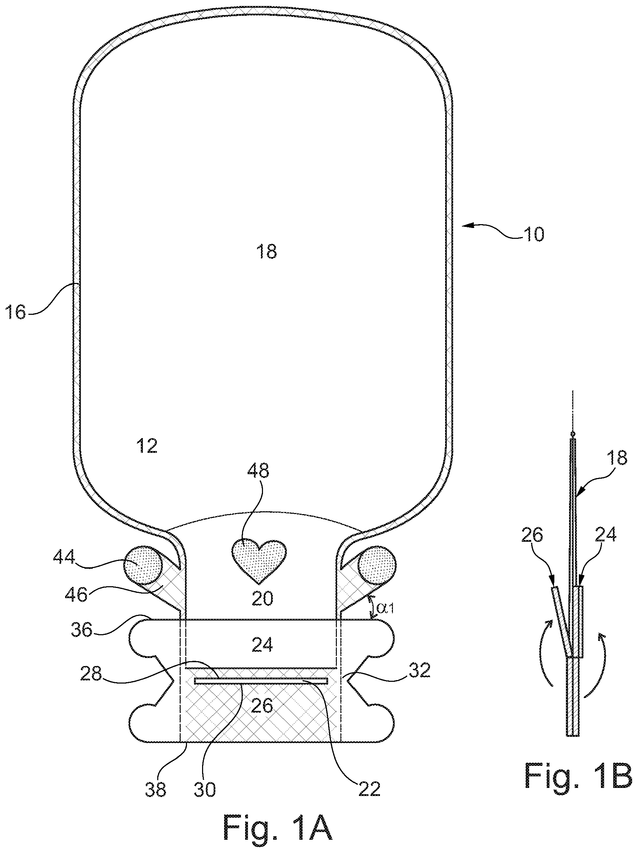

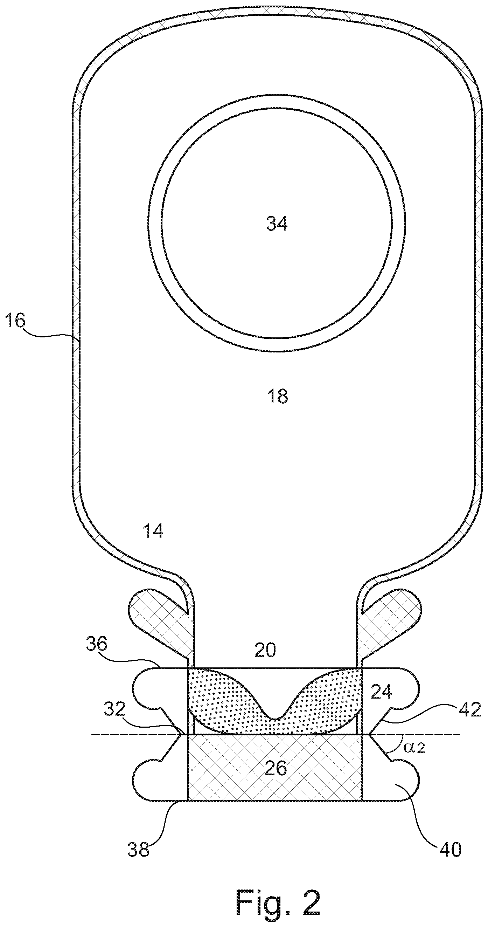

Referring to FIGS. 1A, 1B and 2 of the drawings, the numeral 10 designates a drainable ostomy bag having generally parallel sidewalls of flexible sheet material in the form of a front wall 12 and a rear wall 14. The front wall 12 is facing away from the skin. The sidewalls 12 and 14 are joined along their edges as at 16 to define a cavity 18 there between. They also define a downwardly-extending neck portion 20 which terminates in a discharge opening 22. The bag can be closed by folding the neck portion 20 upwardly and opened by unfolding the neck portion 20 downwardly for draining the contents from the cavity 18.

The discharge opening 22 is bordered with a first and a second plate 24 and 26, each plate 24, 26 comprising at least a first edge portion 28,30 and a second edge 36,38 portion. The first and the second plates 24, 26 are connected by a two strap members 32 at the edge of the discharge opening, thereby surrounding the discharge opening 22. The plates 24, 26 are attached to the opening 22 of the bag along the first edge portion 28, 30 enabling the plates to be pivoted, without folding the neck portion, approximately 180 degrees around their attachment line 28, 30 (being the first edge) with respect to the neck portion 20.

With the arrangement illustrated in FIGS. 1A, B and 2, the sidewalls 12, 14 are formed of a flexible plastic film that is substantially water and gas impermeable. One of the sidewalls 14 (the rear or body side wall) is provided with a stoma-receiving, waste inlet opening 34 surrounded by means for attaching the drainable ostomy bag 10 to a patient. The attaching means may take the form of a coupling device provided for detachably securing the drainable bag 10 to an adhesive faceplate/wafer which together define a two-piece appliance. Alternatively, the bag may be a one-piece appliance with an integral and non-separable faceplate/wafer.

The second edge of the plates 36, 38 may be longer than the first edge 28, 30. The second edges 36, 38 are longer than the width of the discharge opening 22 and extend into a tab member 40. The first 28,30 and the second edges 36,38 are connected with a sloping edge line 42, the line defines an angle α2 with the discharge opening 22/first edge 28,30.

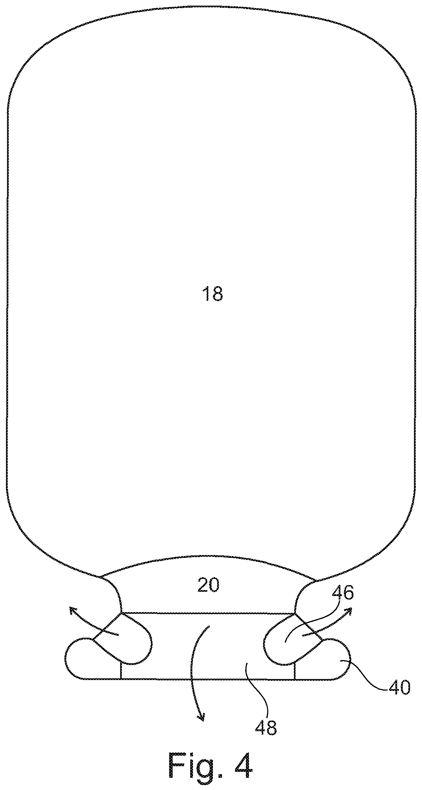

Referring once again to FIGS. 1A and 1B, the drainable ostomy bag will also be seen to include a two-part fastener system for selectively holding the neck portion 20 in upwardly folded condition as best shown in Fig. 4. The two-part fastener system includes locking means in the form of a first part 44 associated with one of the fastening strips 46 extending from the neck portion 20. A second part 48 is provided on the rear wall of the neck portion or on the second first surface of the plate and engages the first part of the locking means when the fastening strip 46 is bended around the folded neck portion. The fastening strips 46 are defining an angle α1 with the discharge opening 22 corresponding substantially to the angle of the sloping edge line of the plates.

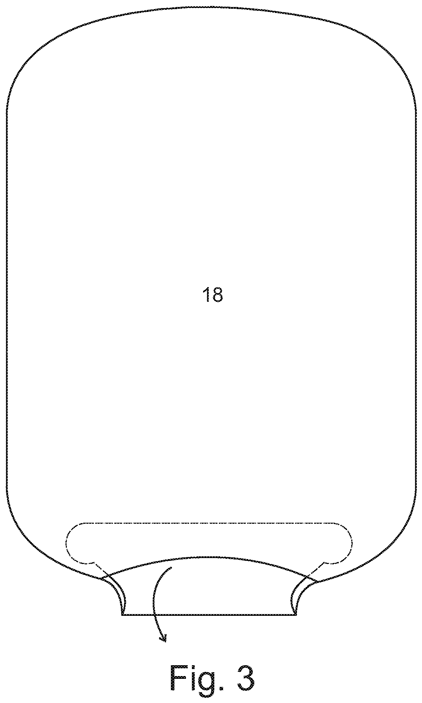

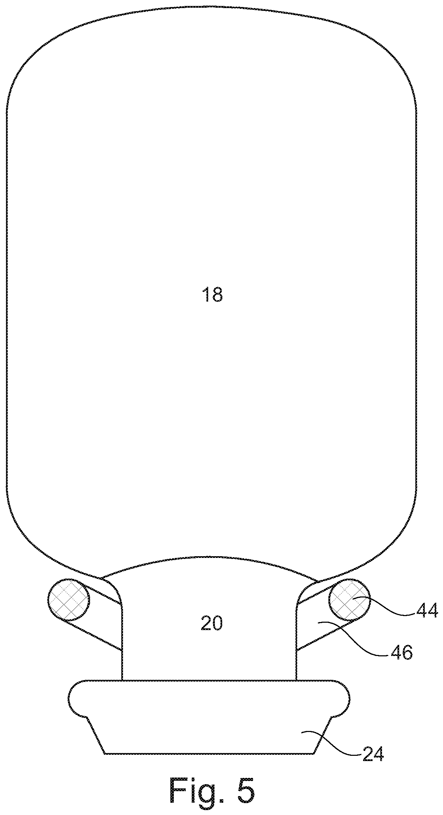

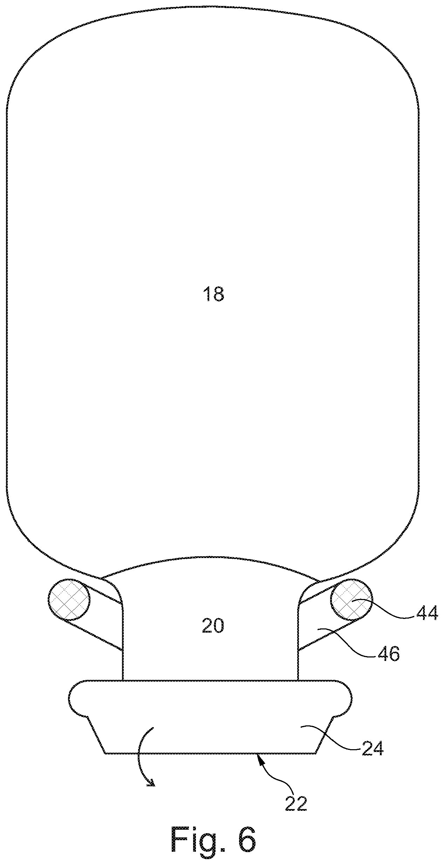

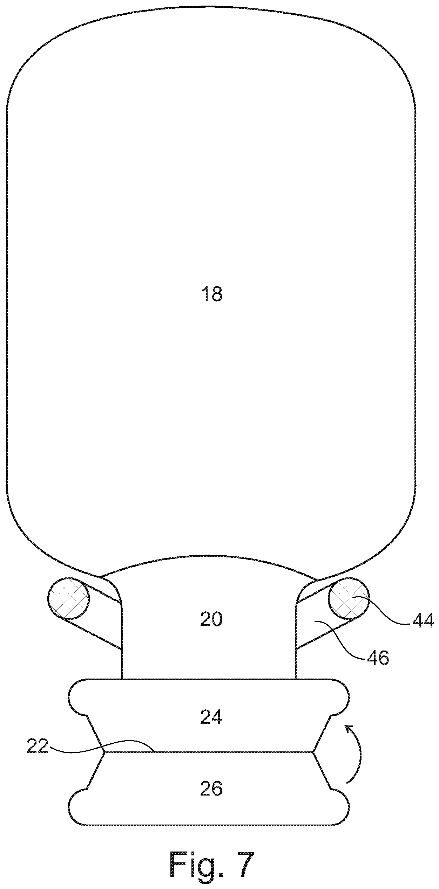

In Figs. 3-7 is shown the unfolding of the neck portion 20 from a closed configuration to an open configuration. The neck portion can be folded up into a closed configuration by facing the first surface of the first plate towards the first surface of the second plate, holding the two surfaces firmly together while folding the neck portion around them, thereby providing a liquid tight seal. When the neck portion is folded up, the fastening strips are engaged to be fixed around the folded neck portion/plates and attaching them to the neck portion. The slanting fastening strips provide better fixation of the folded/rolled neck portion by crossing the folding. After the folded neck portion is fixed by the strips it may be folded one more time and hidden behind the cover layer of the bag as shown in Figure 3.

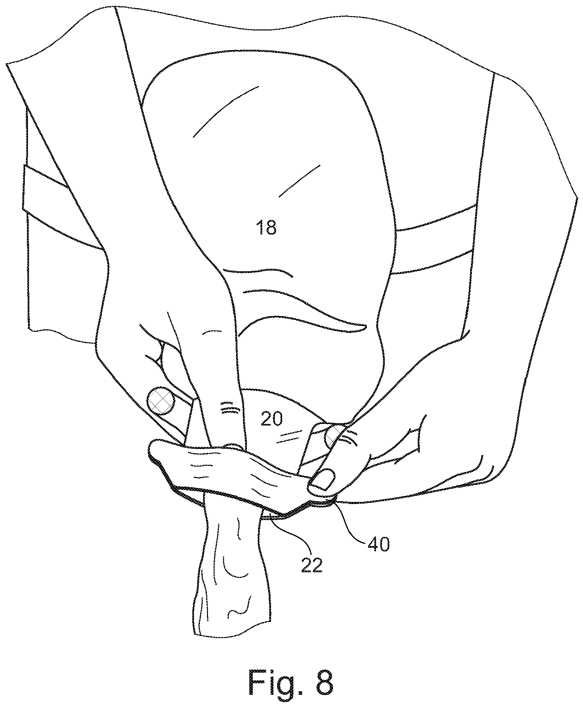

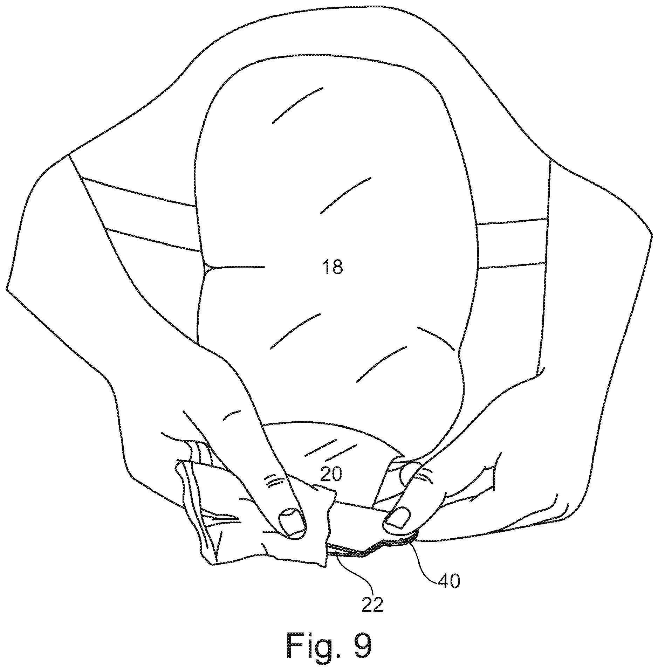

When the bag needs to be emptied, the fastening strips 46 are detached, and the neck portion 20 is unfolded/unrolled. The plates are folded back to bring second surfaces together (with the neck portion in between) and the content of the bag is poured/squeezed out of the bag, holding the extended portion 40 of the plates together, see Figure 8. The last residues of content can be pressed out by putting the fingers between the plate and the neck portion and squeeze. The fingers are protected from contamination by the plate and the construction allows for total emptying of the bag as the entire outer surface of the neck portion can be accessed without getting fingers greased by holding the tab members which extend further than the width of the neck portion. The discharge closure is then wiped clean while holding the tab member, Figure 9.

While in the foregoing there have been set forth preferred embodiments of the invention, it will be appreciated that the details herein given may be varied by those skilled in the art without departing from the true scope of the appended claims.

## Claims

1. A drainable collecting bag (10) for human body wastes, comprising
- an adhesive wafer comprising a waste inlet opening,
- a front wall (12) and a rear wall (14) of flexible sheet material defining a cavity (18) there between and which define a downwardly extending neck portion (20) terminating in a discharge opening (22), a first and a second plate member (24, 26), the first and the second plate members comprising at least a first edge portion (28, 30) and a second edge portion (36, 38), the first plate member being attached to the front wall (12) and the second plate member attached to the rear wall (14), the first edge of the first plate member (28) and the first edge of the second plate member (30) are facing each other and said discharge opening extending between said edges, wherein the first and the second plates (24, 26) can pivot 180 degrees around the first edge portion (28) with respect to the front and the rear walls, the first and the second plate (24, 26) are non-adhesive **characterized in that** the second edge portions (36, 38) of the plate members (24, 26) have a length extending further than the width of the discharge opening (22).

2. Collecting bag according to claim 1, wherein the first edge portions (28, 30) of the plate members (24, 26) have a length substantially corresponding to the width of the discharge opening (22).

3. Collecting bag according any of the preceding claims, wherein the first and the second plate members (24, 26) are connected by at least two strap members (32).

4. Collecting bag according to claim 3, wherein said strap members (32) are formed integrally with the plate members (24, 26), said strap members (32) and plate members (24, 26) forming a unit.

5. Collecting bag according any of the preceding claims, wherein the neck portion (20) is configured to be folded upwardly to a closed configuration.

6. Collecting bag according any of the preceding claims, wherein the first and the second edge portions (28, 30) of the plate members (24, 26) are substantially parallel.

7. Collecting bag according any of the preceding claims, wherein the bag is provided with fastening strips (46).

8. Collecting bag according claim 7, wherein the fastening strips (46) are located perpendicular to the neck portion (20).

9. Collecting bag according any of claims 7-8, wherein the fastening strips (46) are provided in an angle of 75-15 degrees to the neck portion (20).

10. Collecting bag according any of claims 7-9, wherein fastening strips (46) are pointing towards the bag.

11. Collecting bag according any of the preceding claims, wherein the first and the second edge portions (28, 30, 36, 38) are connected with a sloping edge portion.

12. Collecting bag according claim 11, wherein the sloping edge portion defines an angle corresponding to the angle of the fastening strips (46).

13. Collecting bag according any of the preceding claims, wherein the first and the second plates (24, 26) are provided with a tab member (40).

## Patentansprüche

1. Entleerbarer Sammelbeutel (10) für menschliche Körperabfälle, umfassend
- ein Klebeplättchen, das eine Abfalleinlassöffnung umfasst,
- eine Vorderwand (12) und eine Rückwand (14) aus einem flexiblen Flächenmaterial, die einen Hohlraum (18) zwischen sich definieren und die einen sich nach unten erstreckenden Halsabschnitt (20) definieren, der in einer Abgabeöffnung (22) endet,
- ein erstes und ein zweites Plattenelement (24, 26), wobei die ersten und zweiten Plattenelemente mindestens einen ersten Randabschnitt (28, 30) und einen zweiten Randabschnitt (36, 38) umfassen, wobei das erste Plattenelement an der Vorderwand (12) befestigt ist und das zweite Plattenelement an der Rückwand (14) befestigt ist, wobei der erste Rand des ersten Plattenelement (28) und der erste Rand des zweiten Plattenelements (30) einander zugewandt sind und sich die Abgabeöffnung zwischen den Rändern erstreckt, wobei die ersten und zweiten Platten (24, 26) um 180 Grad um den ersten Randabschnitt (28) bezüglich der Vorderwand und der Rückwand schwenken können, wobei die erste und die zweite Platte (24, 26) nicht klebend sind, **dadurch gekennzeichnet, dass** die zweiten Randabschnitte (36, 38) der Plattenelemente (24, 26) eine Länge aufweisen, die sich weiter erstreckt als die Breite der Abgabeöffnung (22).

2. Sammelbeutel nach Anspruch 1, wobei die ersten Randabschnitte (28, 30) der Plattenelemente (24, 26) eine Länge aufweisen, die im Wesentlichen der Breite der Abgabeöffnung (22) entspricht.

3. Sammelbeutel nach einem der vorhergehenden Ansprüche, wobei die ersten und zweiten Plattenelemente (24, 26) durch mindestens zwei Riemenelemente (32) miteinander verbunden sind.

4. Sammelbeutel nach Anspruch 3, wobei die Riemenelemente (32) einstückig mit den Plattenelementen (24, 26) ausgebildet sind, wobei die Riemenelemente (32) und die Plattenelemente (24, 26) eine Einheit bilden.

5. Sammelbeutel nach einem der vorhergehenden Ansprüche, wobei der Halsabschnitt (20) dazu ausgelegt ist, nach oben in eine geschlossene Konfiguration gefaltet zu werden.

6. Sammelbeutel nach einem der vorhergehenden Ansprüche, wobei die ersten und zweiten Randabschnitte (28, 30) der Plattenelemente (24, 26) im Wesentlichen parallel sind.

7. Sammelbeutel nach einem der vorhergehenden Ansprüche, wobei der Beutel Befestigungsstreifen (46) aufweist.

8. Sammelbeutel nach Anspruch 7, wobei die Befestigungsstreifen (46) senkrecht zum Halsabschnitt (20) angeordnet sind.

9. Sammelbeutel nach einem der Ansprüche 7-8, wobei die Befestigungsstreifen (46) in einem Winkel von 75-15 Grad zum Halsabschnitt (20) vorgesehen sind.

10. Sammelbeutel nach einem der Ansprüche 7-9, wobei die Befestigungsstreifen (46) zum Beutel gerichtet sind.

11. Sammelbeutel nach einem der vorhergehenden Ansprüche, wobei die ersten und zweiten Randabschnitte (28, 30, 36, 38) mit einem geneigten Randabschnitt verbunden sind.

12. Sammelbeutel nach Anspruch 11, wobei der geneigte Randabschnitt einen Winkel definiert, der dem Winkel der Befestigungsstreifen (46) entspricht.

13. Sammelbeutel nach einem der vorhergehenden Ansprüche, wobei die ersten und zweiten Platten (24, 26) mit einem Laschenelement (40) ausgestattet sind.

## Revendications

1. Sac collecteur vidangeable (10) pour déchets d'origine humaine, comprenant
- une pastille adhésive comprenant une ouverture d'entrée pour les déchets,
- une paroi avant (12) et une paroi arrière (14) en matériau en feuille flexible définissant une cavité (18) entre elles, et qui définissent une partie de col s'étendant vers le bas (20) se terminant par une ouverture de décharge (22), un premier et un deuxième organe de plaque (24, 26), le premier et le deuxième organe de plaque comprenant au moins une première partie de bord (28, 30) et une deuxième partie de bord (36, 38), le premier organe de plaque étant attaché à la paroi avant (12) et le deuxième organe de plaque étant attaché à la paroi arrière (14), le premier bord du premier organe de plaque (28) et le premier bord du deuxième organe de plaque (30) se faisant face et ladite ouverture de décharge s'étendant entre lesdits bords, la première et la deuxième plaque (24, 26) pouvant pivoter de 180 degrés autour de la première partie de bord (28) par rapport à la paroi avant et la paroi arrière, la première et la deuxième plaque (24, 26) étant non-adhésives, **caractérisé en ce que** les deuxièmes parties de bord (36, 38) des organes de plaque (24, 26) ont une longueur s'étendant plus loin que la largeur de l'ouverture de décharge (22).

2. Sac collecteur selon la revendication 1, dans lequel les premières parties de bord (28, 30) des organes de plaque (24, 26) ont une longueur correspondant sensiblement à la largeur de l'ouverture de décharge (22).

3. Sac collecteur selon l'une quelconque des revendications précédentes, dans lequel le premier et le deuxième organe de plaque (24, 26) sont connectés par au moins deux organes de bande (32).

4. Sac collecteur selon la revendication 3, dans lequel lesdits organes de bande (32) sont formés intégralement avec les organes de plaque (24, 26), lesdits organes de bande (32) et les organes de plaque (24, 26) formant une unité.

5. Sac collecteur selon l'une quelconque des revendications précédentes, dans lequel la portion de col (20) est configurée pour être pliée vers le haut dans une configuration fermée.

6. Sac collecteur selon l'une quelconque des revendications précédentes, dans lequel la première et la deuxième partie de bord (28, 30) des organes de plaque (24, 26) sont sensiblement parallèles.

7. Sac collecteur selon l'une quelconque des revendications précédentes, dans lequel le sac est pourvu de rubans d'attache (46).

8. Sac collecteur selon la revendication 7, dans lequel les rubans d'attache (46) sont situés perpendiculairement à la partie de col (20).

9. Sac collecteur selon l'une quelconque des revendications 7 à 8, dans lequel les rubans d'attache (46) sont disposés à un angle de 75-15 degrés par rapport à la portion de col (20).

10. Sac collecteur selon l'une quelconque des revendications 7 à 9, dans lequel les rubans d'attache (46) pointent vers le sac.

11. Sac collecteur selon l'une quelconque des revendications précédentes, dans lequel la première et la deuxième partie de bord (28, 30, 36, 38) sont connectées par une partie de bord inclinée.

12. Sac collecteur selon la revendication 11, dans lequel la partie de bord inclinée définit un angle correspondant à l'angle des rubans d'attache (46).

13. Sac collecteur selon l'une quelconque des revendications précédentes, dans lequel la première et la deuxième plaque (24, 26) sont pourvues d'un organe de languette (40).
